(19) Europäisches Patentamt
European Patent Office
Office européen des brevets

(11) Veröffentlichungsnummer: **0 039 037**
**B1**

(12) # EUROPÄISCHE PATENTSCHRIFT

(45) Veröffentlichungstag der Patentschrift: **16.01.85**

(51) Int. Cl.⁴: **C 07 D 239/52**, C 07 D 239/46

(21) Anmeldenummer: **81102997.4**

(22) Anmeldetag: **18.04.81**

(54) **Pyrimidylthioharnstoffe, diese enthaltende Arzneimittel und deren Verwendung.**

(30) Priorität: **30.04.80 DE 3016767**

(43) Veröffentlichungstag der Anmeldung: **04.11.81 Patentblatt 81/44**

(45) Bekanntmachung des Hinweises auf die Patenterteilung: **16.01.85 Patentblatt 85/3**

(84) Benannte Vertragsstaaten: **AT BE CH DE FR GB IT LI LU NL SE**

(56) Entgegenhaltungen:
**DE - A - 2 849 537**

**CHEMICAL ABSTRACTS, Band 72, 1970, Seite 261, Nr. 31191d, Columbus, Ohio, U.S.A., V. BAEVA et al.: "Synthesis of thiourea derivatives"**

**Die Akte enthält technische Angaben, die nach dem Eingang der Anmeldung eingereicht wurden und die nicht in dieser Patentschrift enthalten sind.**

(73) Patentinhaber: **Beiersdorf Aktiengesellschaft, Unnastrasse 48, D-2000 Hamburg 20 (DE)**

(72) Erfinder: **Stenzel, Wolfgang, Dr. Dipl.-Chem, Lerchenweg 8, D-2057 Reinbek (DE)**

**EP 0 039 037 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

## Beschreibung

Die Erfindung bezieht sich auf neue substituierte Pyrimidylthioharnstoffe der allgemeinen Formel I

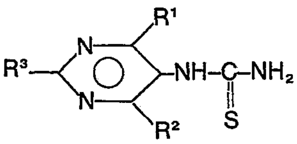

(I)

in der die Reste $R^1$, $R^2$ und $R^3$, die gleich oder verschieden sein können, ein Wasserstoff- oder Halogenatom, eine geradkettige oder verzweigte Alkoxy- oder Alkylgruppe mit jeweils 1 bis 8, vorzugsweise 1 bis 4 Kohlenstoffatomen bedeuten, und deren physiologisch verträglichen Salze, diese Verbindungen enthaltende Arzneimittel und deren Verwendung.

Bevorzugt werden Verbindungen der allgemeinen Formel I, in der $R^1$, $R^2$ und $R^3$ Alkoxy und/oder Alkylgruppen bedeuten. Besonders bevorzugte Alkoxygruppen sind die Methoxygruppe und die Äthoxygruppe, insbesondere die Methoxygruppe und besonders bevorzugte Alkylgruppen sind die Methylgruppe und die Äthylgruppe, insbesondere die Methylgruppe. Vorzugsweise sind zwei der Substituenten Alkoxygruppen und der dritte Substituent ist Wasserstoff oder eine Alkylgruppe.

Einige Verbindungen der Formel I wurden als Zwischenprodukte für die Herstellung von 5-(2-Imidazolin-2-yl)-aminopyrimidinen verwendet. Es sind die in der DE-A-2 849 537 genannten Verbindungen N-(4,6-Dimethoxy-5-pyrimidyl)-thioharnstoff, N-(2,4-Dimethoxy-5-pyrimidyl)-thioharnstoff und N-(4,6-Diäthoxy-5-pyrimidyl)-thioharnstoff. Die Verbindungen der Formel I, worin die Reste $R^1$, $R^2$ und $R^3$, die gleich oder verschieden sein können, ein Wasserstoff- oder Halogenatom, eine geradkettige oder verzweigte Alkoxy- oder Alkylgruppe mit 5 bis 8 Kohlenstoffatomen bedeuten, wurden nicht verwendet. Bevorzugt werden niederhalogenierte und insbesondere monochlorierte Pyrimidylthioharnstoffderivate.

Es wurde nunmehr völlig überraschend festgestellt, dass die Verbindungen der allgemeinen Formel I eine hohe pharmakologische Wirkung besitzen und als Arzneimittel, insbesondere als Lipidsenker und/oder Antihypertensiva, verwendet werden können.

Die Verbindungen der Formel I zeigen an hyperlipidämischen Mäusen bei einer Verabreichungsmenge von 1 bis 300 mg/kg eine cholesterin- und triglyceridsenkende Wirkung.

Die Verbindungen gemäss der Erfindung besitzen gegenüber dem bekannten Therapeuticum (Clofibrat) α-(p-Chlorphenoxy)isobuttersäureäthylester und ähnlichen bekannten Lipidsenkern den therapeutischen Vorteil, dass sie eine wesentlich höhere Wirkungsstärke besitzen und ausserdem keine toxischen Eigenschaften aufweisen. Dies gilt insbesondere für die Verbindungen der Formel I, bei welchen $R^1$ und $R^2$ eine Alkoxygruppe und $R^3$ ein Wasserstoffatom bedeuten.

Einige Verbindungen der Formel I zeichnen sich durch wertvolle blutdrucksenkende Eigenschaften aus. Bei Ratten mit hohem Blutdruck tritt dieser therapeutische Effekt bei einer Verabreichungsmenge im Bereich von 1 bis 30 mg/kg auf, wobei keine zentralen Nebenwirkungen in Erscheinung treten. Die wegen störender Begleitwirkungen unerwünschte α-Rezeptoren-Blockade ist ebenfalls nicht vorhanden.

Die Verbindungen der Formel I sind daher gemäss der Erfindung zur Behandlung von Hyperlipidämie und/oder Krankheiten des Herz-Kreislaufsystems, vorzugsweise Hypertonie, geeignet. Die Verabreichungsmenge je Tag liegt im Bereich von 10 bis 1000 mg, wobei die Verabreichung zweckmässig in Teilmengen von 5 bis 500 mg zwei- bis viermal täglich oder in einer verzögerten Abgabeform nur einmal täglich erfolgt. Eine bevorzugte Verabreichungs-Tagesdosis liegt im Bereich von etwa 50 bis 300 mg.

Durch geeignete Wahl der Substituenten $R^1$, $R^2$ und $R^3$ kann die therapeutische Hauptwirkung so beeinflusst werden, dass entweder die antilipidämischen oder blutdrucksenkenden Eigenschaften überwiegen.

Beispiele für Verbindungen der allgemeinen Formel I mit einem therapeutischen Effekt sind nachstehend aufgeführt.

N-(4,6-Dimethoxy-6-pyrimidyl)-thioharnstoff
N-(4-Äthoxy-6-methoxy-6-pyrimidyl)-thioharnstoff
N-(4-Isopropoxy-6-methoxy-5-pyrimidyl)-thioharnstoff
N-(4-Butoxy-6-methoxy-5-pyrimidyl)-thioharnstoff
N-(4,6-Diäthoxy-5-pyrimidyl)-thioharnstoff
N-(4,6-Dibutoxy-5-pyrimidyl)-thioharnstoff
N-(4-Methoxy-6-methyl-5-pyrimidyl)-thioharnstoff
N-(4,6-Dimethyl-5-pyrimidyl)-thioharnstoff
N-(2,4-Dimethoxy-5-pyrimidyl)-thioharnstoff
N-(2-Chlor-4-methyl-5-pyrimidyl)-thioharnstoff
N-(2-Äthoxy-4-methoxy-5-pyrimidyl)-thioharnstoff
N-(2,4-Diäthoxy-5-pyrimidyl)-thioharnstoff
N-(2,4-Diisopropoxy-5-pyrimidyl)-thioharnstoff
N-(4-Methoxy-2-methyl-5-pyrimidyl)-thioharnstoff
N-(2-Methyl-4-propoxy-5-pyrimidyl)-thioharnstoff
N-(2-Methyl-5-pyrimidyl)-thioharnstoff
N-(2-Isopropyl-5-pyrimidyl)-thioharnstoff
N-(4-Methyl-5-pyrimidyl)-thioharnstoff
N-(4-Äthyl-5-pyrimidyl)-thioharnstoff
N-(4-Butyl-5-pyrimidyl)-thioharnstoff
N-(2-Methoxy-5-pyrimidyl)-thioharnstoff
N-(4-Methoxy-5-pyrimidyl)-thioharnstoff
N-(4-Äthoxy-5-pyrimidyl)-thioharnstoff
N-(4-Propoxy-5-pyrimidyl)-thioharnstoff
N-(4-Isopropoxy-5-pyrimidyl)-thioharnstoff
N-(4-Butoxy-5-pyrimidyl)-thioharnstoff
N-(5-Pyrimidyl)-thioharnstoff

N-(2,4,6-Trimethoxy-5-pyrimidyl)-thioharnstoff
N-(2,4,6-Trimethyl-5-pyrimidyl)-thioharnstoff
N-(2,4-Dimethoxy-6-methyl-5-pyrimidyl)-thioharnstoff
N-(2,4-Diäthoxy-6-methyl-5-pyrimidyl)-thioharnstoff
N-(4,6-Dimethoxy-2-methyl-5-pyrimidyl)-thioharnstoff
N-(4,6-Dimethoxy-2-isopropyl-5-pyrimidyl)-thioharnstoff
N-(4-Äthoxy-6-methoxy-2-methyl-5-pyrimidyl)-thioharnstoff
N-(4-Äthoxy-2-äthyl-6-methoxy-5-pyrimidyl)-thioharnstoff
N-(2-Butyl-4-methoxy-6-propoxy-5-pyrimidyl)-thioharnstoff
N-(2-Chlor-4-methyl-6-methoxy-5-pyrimidyl)-thioharnstoff
N-(2-Chlor-4-propyl-6-isopropoxy-5-pyrimidyl)-thioharnstoff
N-(4-Chlor-6-methoxy-2-methyl-5-pyrimidyl)-thioharnstoff
N-(4-Chlor-6-äthoxy-2-isopropyl-5-pyrimidyl)-thioharnstoff
N-(4-Methoxy-6-pentyloxy-5-pyrimidyl)-thioharnstoff
N-(4-Methoxy-2-tert.-pentyl-5-pyrimidyl)-thioharnstoff
N-(2-Methoxy-4-pentyloxy-5-pyrimidyl)-thioharnstoff
N-(4,6-Di-hexyloxy-5-pyrimidyl)-thioharnstoff
N-(2-Heptyl-4-methoxy-5-pyrimidyl)-thioharnstoff
N-(4-Heptyloxy-2-pentyl-5-pyrimidyl)-thioharnstoff
N-(2-Chlor-4-methyl-6-octyloxy-5-pyrimidyl)-thioharnstoff
N-(2-Octyl-4-methyl-6-heptoxy-5-pyrimidyl)-thioharnstoff
N-(2-Brom-4-methyl-6-methoxy-5-pyrimidyl)-thioharnstoff

Besonders bevorzugte Verbindungen mit einem hohen therapeutischen Effekt sind a) N-(4,6-Dimethoxy-5-pyrimidyl)-thioharnstoff, b) N-(2,4-Dimethoxy-5-pyrimidyl)-thioharnstoff, c) N-(4,6-Dimethoxy-2-methyl-5-pyrimidyl)-thioharnstoff und d) N-(4,6-Diäthoxy-5-pyrimidyl)-thioharnstoff. Diese Verbindungen besitzen eine lipidsenkende und/oder blutdrucksenkende Wirkung.

Gemäss der Erfindung werden pharmazeutische Zusammensetzungen geschaffen, die eine Verbindung der Formel I oder deren pharmazeutisch verträglichen Salze, zusammen mit einem pharmazeutisch verträglichen Verdünnungsmittel oder Träger enthalten.

Die Verbindungen gemäss der Erfindung können mit üblichen pharmazeutisch verträglichen Verdünnungsmitteln oder Trägern und gegebenenfalls mit anderen Hilfsmitteln vermischt und beispielsweise oral oder parenteral verabreicht werden. Sie können oral in Form von Tabletten, Dragees, Sirups, Suspensionen und Flüssigkeiten, oder parenteral in Form von Lösungen oder Suspensionen verabreicht werden. Oral zu verabreichende Präparate können ein oder mehrere Zusätze, wie Süssungsmittel, Aromatisierungsmittel, Farbstoffe und Konservierungsmittel, enthalten. Tabletten können den Wirkstoff mit üblichen, pharmazeutisch verträglichen Hilfsmitteln vermischt enthalten, z.B. inerten Verdünnungsmitteln wie Calciumcarbonat, Natriumcarbonat, Lactose und Talk, Granulierungsmitteln und Mitteln, die den Zerfall der Tabletten bei oraler Verabreichung fördern, wie Stärke oder Alginsäure, Bindemitteln wie Stärke oder Gelatine, Gleitmitteln wie Magnesiumstearat, Stearinsäure und Talk.

Die Tabletten können nach bekannten Arbeitsweisen so überzogen werden, um den Zerfall und die Resorption im Magen-Darmtrakt zu verzögern, wodurch die Aktivität des Wirkstoffs sich über eine längere Zeitspanne erstrecken kann. Ebenso kann in den Suspensionen der Wirkstoff mit Hilfsmitteln vermischt sein, die für die Herstellung solcher Zusammensetzungen üblich sind, z.B. Suspendiermitteln wie Methylcellulose, Tragacanth oder Natriumalginat, Netzmitteln wie Lecithin, Polyoxyäthylenstearat und Polyoxyäthylensorbitanmonooleat, und Konservierungsmitteln wie Äthylparahydroxybenzoat. Kapseln können den Wirkstoff als einzigen Bestandteil oder vermischt mit einem festen Verdünnungsmittel wie Calciumcarbonat, Calciumphosphat oder Kaolin enthalten. Die injizierbaren Präparate werden ebenfalls in an sich bekannter Weise formuliert. Die pharmazeutischen Präparate können den Wirkstoff in einer Menge von 0,1 bis 90%, insbesondere 1 bis 90%, enthalten, wobei der Rest ein Trägerstoff oder Zusatzstoff ist. Im Hinblick auf die Herstellung und Verabreichung werden feste Präparate, wie Tabletten und Kapseln, bevorzugt. Vorzugsweise enthalten die Präparate den Wirkstoff in einer Menge von 50 bis 300 mg entsprechend der bevorzugten Tagesdosis.

Die Verbindungen der Formel I können aus Aminopyrimidinen der nachstehenden Formel II

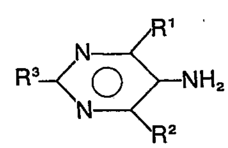

(II)

worin die Reste $R^1$, $R^2$ und $R^3$ die vorstehend angegebene Bedeutung besitzen, durch Umsetzung mit Benzoylisothiocyanat, das aus Ammoniumthiocyanat und Benzoylchlorid erhältlich ist, hergestellt werden. Dabei entstehen die entsprechenden Benzoylthioharnstoffe, die zu den Pyrimidylthioharnstoffen der Formel I hydrolysiert werden können.

Die Bildung der Pyrimidylbenzoylthioharnstoffe erfolgt zweckmässig durch Umsetzung von Ammoniumthiocyanat, Benzoylchlorid und Aminopyrimidin in einem organischen Lösungsmittel unter Erwärmen, vorzugsweise in siedendem Aceton. Die Umsetzung wird vorzugsweise im Bereich

von Raumtemperatur bis zur Siedetemperatur des verwendeten Lösungsmittels während einer Dauer von 30 min bis 3 h, vorzugsweise 1 h, durchgeführt. Das erhaltene Reaktionsgemisch wird dann in Wasser eingebracht und das Reaktionsprodukt mit einem mit Wasser nicht mischbaren Lösungsmittel, vorzugsweise Chloroform, extrahiert. Die erhaltenen Thioharnstoff-Derivate werden dann zur Abspaltung des Benzoylrestes beispielsweise in Gegenwart von Basen, gegebenenfalls unter Erwärmen, hydrolysiert. Als Basen sind verdünnte Natronlauge oder Natriummethylatlösung geeignet.

In Thioharnstoffderivate der Formel I, in denen $R^1$ und $R^2$ eine Alkoxygruppe, vorzugsweise eine Methoxygruppe, bedeuten und $R^3$ die vorstehend angegebene Bedeutung besitzt, kann durch Umsetzung mit einem entsprechenden Alkoholat ein neuer Alkoxyrest eingeführt werden.

Man führt die Umsetzung vorzugsweise mit äquimolaren Mengen an Natriumalkoholat in einem dem Alkoholat entsprechenden Alkohol bei erhöhter Temperatur, vorzugsweise bei der Siedetemperatur des Alkohols, durch. Dabei erhält man die Verbindungen der Formel I, worin die Reste $R^1$, $R^2$ Alkoxyreste bedeuten, die voneinander verschieden sein können.

Die für die Herstellung der Verbindungen der Formel I verwendeten Aminopyrimidine der Formel II sind entweder bekannt oder in an sich bekannter Weise aus verfügbaren Ausgangsstoffen herstellbar (vgl. D.J. Brown, «The Pyrimidines», 1962, Interscience Publishers).

Alkoxypyrimidine können aus Chlorpyrimidinen durch Umsetzung mit Alkoholat in einem geeigneten Lösungsmittel, vorzugsweise in einem dem Alkoholat entsprechenden Alkohol, hergestellt werden. Sind mehrere Chloratome am Pyrimidinkern vorhanden, können durch geeignete Auswahl der Reaktionsbedingungen, insbesondere der Konzentration des Alkoholats und der Reaktionstemperatur, entweder ein oder auch mehrere Halogenatome gegen die entsprechenden Alkoxygruppen mit 1 bis 8, vorzugsweise 1 bis 4 Kohlenstoffatomen ausgetauscht werden. Es können auch nacheinander verschiedene Alkoxygruppen eingeführt werden. Diese Substitutionsreaktionen sind im allgemeinen sowohl bei halogensubstituierten 5-Nitropyrimidinen als auch bei halogensubstituierten 5-Aminopyrimidin-Derivaten durchführbar.

Die an den Pyrimidinring gebundenen Chloratome können durch katalytische Hydrierung in Gegenwart einer Base abgespalten werden. Die Entfernung von Chloratomen kann auch durch Umsetzung entsprechender Chlorpyrimidine mit Hydrazin und anschliessende Abspaltung der Hydrazingruppe mit Silber(I)-oxyd durchgeführt werden. Die Umsetzung mit Hydrazin wird zweckmässig in einem geeigneten Lösungsmittel vorzugsweise Methanol oder Äthanol, bei Temperaturen zwischen 0 °C und der Siedetemperatur des Lösungsmittels durchgeführt.

Die Umsetzung mit Silber(I)-oxyd kann in einem Lösungsmittel wie Methanol oder Äthanol bei Raumtemperatur oder erhöhter Temperatur durchgeführt werden.

Chlornitropyrimidine der allgemeinen Formel III

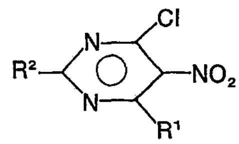

(III)

in der $R^1$ und $R^2$, die gleich oder verschieden sein können, eine geradkettige oder verzweigte Alkyl- oder Alkoxygruppe mit 1 bis 8 Kohlenstoffatomen, ein Wasserstoffatom oder Chloratom bedeuten, können aus Hydroxypyrimidinen der Formel IV

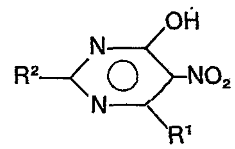

(IV)

in der $R^1$ und $R^2$ eine Hydroxy-, Alkyl- oder Alkoxygruppe mit 1 bis 8 Kohlenstoffatomen oder ein Wasserstoffatom bedeuten, durch Umsetzung mit einem geeigneten Chlorierungsmittel erhalten werden. Als Chlorierungsmittel verwendet man vorzugsweise Phosphoroxytrichlorid in Gegenwart eines tertiären Amins wie Diäthylanilin. Die Umsetzungen werden vorzugsweise ohne Lösungsmittel bei erhöhter Temperatur durchgeführt.

Die geeignet substituierten Nitropyrimidine können durch katalytische Hydrierung in Gegenwart von Raney-Nickel bei einem Druck im Bereich von 1 bis 10 bar in die Aminopyrimidine der Formel II übergeführt werden. Als Lösungsmittel können niedere Alkohole, vorzugsweise Methanol, verwendet werden. Die Reaktionstemperatur liegt zwischen Raumtemperatur und 50 °C.

Die folgenden Beispiele veranschaulichen die Herstellung von Verbindungen gemäss der Erfindung.

Herstellungsbeispiel 1

N-(4,6-Dimethoxy-5-pyrimidyl)-thioharnstoff

(a) 200 g 4,6-Dihydroxypyrimidin werden bei 15 bis 20 °C in ein Gemisch aus 720 ml Eisessig und 230 ml Salpetersäure (96%) eingebracht. Die Mischung wird 30 min lang gerührt, worauf sie auf Eis gegossen wird. Der gebildete Niederschlag wird abgesaugt und mit wenig Eiswasser gewaschen und anschliessend bei 80 °C getrocknet.

Ausbeute: 254 g 4,6-Dihydroxy-5-nitropyrimidin. Fp. >300 °C.

(b) 135 g 4,6-Dihydroxy-5-nitropyrimidin werden in 440 ml Phosphoroxytrichlorid eingerührt. Dann fügt man langsam 160 ml N,N-Diäthylanilin hinzu und erhitzt während einer Stunde bei 120 °C. Anschliessend wird das überschüssige Phosphoroxytrichlorid abdestilliert, der Rückstand wird auf Eis gegossen und der dabei erhaltene Niederschlag wird abfiltriert, der Niederschlag wird in wenig Wasser aufgenommen und gerührt, worauf mit Natriumbicarbonat neutralisiert wird. Der

Rückstand wird abgesaugt, mit 500 ml Cyclohexan aufgekocht. Die dabei erhaltene klare Lösung wird vom ungelösten Rückstand zur Isolierung des Reaktionsproduktes abgegossen und eingedampft. Nach Trocknung werden 131 g 4,6-Dichlor-5-nitropyrimidin erhalten. Fp. 103 °C.

(c) In eine Lösung von 62 g NaOH in 1200 ml Methanol werden 120 g 5-Nitro-4,6-dichlorpyrimidin eingebracht. Man erhitzt 1 h unter Rückfluss und gibt die Reaktionsmischung nach dem Abkühlen in 3 l Wasser. Die dabei erhaltenen Kristalle werden abfiltriert und getrocknet. Man erhält 87 g 4,6-Dimethoxy-5-nitropyrimidin. Fp. 168 bis 170 °C.

(d) 125 g 4,6-Dimethoxy-5-nitropyrimidin werden in 1,5 l Äthanol gelöst und in einem Edelstahlautoklaven in Gegenwart von 40 g Raney-Nickel bei 2,5 bar während 5 h hydriert. Der Katalysator wird abfiltriert und das Filtrat eingeengt. Dabei werden 88 g 5-Amino-4,6-dimethoxypyrimidin, Fp. 94 bis 96 °C, erhalten.

(e) In eine Lösung von 12,9 g Ammoniumthiocyanat in 120 ml wasserfreiem Aceton lässt man zunächst 25,3 g Benzoylchlorid langsam zutropfen, erhitzt 5 min unter Rückfluss und tropft dann noch 27 g 5-Amino-4,6-dimethoxypyrimidin, gelöst in 50 ml Aceton, hinzu. Man kocht eine weitere Stunde unter Rückfluss, lässt abkühlen und bringt das Gemisch in 1,5 l Wasser ein. Anschliessend wird mit Chloroform extrahiert, über Magnesiumsulfat getrocknet. Nach Abdestillation des Chloroforms wird der Rückstand mit Äther verrieben und abgesaugt. Ausbeute: 34 g (61%) N-Benzoyl-N′-(4,6-dimethoxy-5-pyrimidyl)-thioharnstoff. Fp. 193 °C.

(f) 34 g des so gewonnenen N-Benzoylthioharnstoffderivats werden in 30 ml 10%iger NaOH während 5 min gekocht. Nach dem Abkühlen wird mit konz. HCl angesäuert und mit 15%iger wässriger Ammoniaklösung auf pH 9 eingestellt. Die ausfallenden Kristalle werden abgesaugt und mit Wasser gewaschen. Es werden 16 g (70%) N-(4,6-Dimethoxy-5-pyrimidyl)-thioharnstoff erhalten. Fp. 214 °C.

Herstellungsbeispiel 2

N-(4-Methoxy-5-pyrimidyl)-thioharnstoff

(a) 100 g 5-Nitro-4,6-dichlorpyrimidin werden in 1000 ml Natriummethylatlösung (16,8 g Natrium) während 10 h auf 50 °C erwärmt. Danach wird das Reaktionsgemisch im Vakuum eingedampft, der Rückstand mit Wasser verrührt und abgesaugt. Anschliessend wird mit Petroläther (Siedebereich 40 bis 60 °C) mehrmals heiss extrahiert. Nach dem Abkühlen werden die Kristalle abgesaugt und getrocknet. Man erhält 46 g 4-Chlor-6-methoxy-5-nitropyrimidin. Fp. 65 °C.

(b) 15 g 4-Chlor-6-methoxy-5-nitropyrimidin werden in 600 ml Äthanol gelöst. Dann tropft man 8,1 ml Hydrazinhydrat in 150 ml Äthanol bei −8 °C hinzu. Nach einer Stunde werden die dabei erhaltenen Kristalle abgesaugt und getrocknet.

Ausbeute: 14,5 g 4-Hydrazino-6-methoxy-5-nitropyrimidin. Fp. 155 °C.

(c) 14 g 4-Hydrazino-6-methoxy-5-nitro-pyrimidin werden in 3,6 l Methanol gelöst und mit 61,9 g frisch hergestelltem Silber(I)-oxyd während 10 h bei 40 °C gerührt. Danach wird filtriert und die Lösung eingedampft. Man erhält 10,5 g 4-Methoxy-5-nitropyrimidin. Fp. 193 bis 195 °C.

(d) 10 g 4-Methoxy-5-nitropyrimidin in 500 ml Methanol werden in Gegenwart von 4,5 g Raney-Nickel bei 2 bar während 2,5 h hydriert. Nach Filtration wird die Lösung eingeengt. Man erhält 8,0 g 5-Amino-4-methoxypyrimidin. Fp. 71 bis 74 °C.

(e) Ähnlich wie im Herstellungsbeispiel 1 wird aus 5-Amino-4-methoxypyrimidin N-Benzoyl-N′-(4-methoxy-5-pyrimidyl)-thioharnstoff hergestellt.

(f) 6,0 g N-Benzoyl-N′-(4-methoxy-5-pyrimidyl)-thioharnstoff werden in 120 ml Natriumäthylatlösung (0,8 g Natrium) gelöst. Nach 1 h wird mit verdünnter Salzsäure neutralisiert. Man engt das Gemisch ein, wäscht den Rückstand mit Wasser und kristallisiert aus Methanol/Wasser um. Man erhält 2,3 g N-(4-Methoxy-5-pyrimidyl)-thioharnstoff. Fp. 182 bis 183 °C (Zers.).

Herstellungsbeispiel 3

N-(4-Äthoxy-6-methoxy-5-pyrimidyl)-thioharnstoff

3,0 g N-(4,6-Dimethoxy-5-pyrimidyl)-thioharnstoff werden mit einer Natriumäthylatlösung (0,317 g Natrium in 30 ml abs. Äthanol) vermischt und während 5 h unter Rückfluss erhitzt. Nach dem Abkühlen wird neutralisiert. Das Reaktionsprodukt wird abgesaugt und säulenchromatographisch auf Kieselgel gereinigt (Laufmittel: Chloroform/Methanol 95 : 5). Man erhält 0,6 g N-(4-Äthoxy-6-methoxy-5-pyrimidyl)-thioharnstoff. Fp. 186 bis 187 °C.

Analog zu den vorstehenden Herstellungsbeispielen wurden auch die in der nachstehenden Tabelle aufgeführten Verbindungen synthetisiert.

| Herstellungsbeispiel | $R^1$ | $R^2$ | $R^3$ | Fp. °C |
|---|---|---|---|---|
| 5 | $OC_2H_5$ | $OC_2H_5$ | H | 242 |
| 6 | $OC_4H_9$ | $OC_4H_9$ | H | 105–106 (Hydrat) |
| 7 | $OCH_3$ | $CH_3$ | $CH_3$ | 212 |
| 8 | $OCH_3$ | $OCH_3$ | $CH_3$ | 228 |
| 9 | $OCH_3$ | H | $OCH_3$ | 173 |
| 10 | $CH_3$ | $OCH_3$ | Cl | 186 (Zers.) |
| 11 | $OCH_3$ | Cl | $CH_3$ | ab 190 (Zers.) |

Die Herstellung von Arzneimitteln unter Verwendung einer Verbindung gemäss der Erfindung wird nachstehend anhand von Beispielen näher erläutert.

Beispiel 1

Herstellung von Tabletten und Kapseln

Tabletten und Kapseln, welche die nachstehend angegebenen Bestandteile enthalten, werden nach bekannten Arbeitsweisen hergestellt. Diese sind für die Behandlung von Hyperlipidämie in Dosierungsmengen von jeweils einer Tablette oder Kapsel zwei- bis viermal täglich geeignet.

| Bestandteile | Gewicht (mg) Tablette | Kapsel |
|---|---|---|
| N-(4,6-Dimethoxy-5-pyrimidyl)-thioharnstoff | 100 | 100 |
| Tragacanth | 10 | – |
| Lactose | 247,5 | 300 |
| Maisstärke | 25 | – |
| Talk | 15 | – |
| Magnesiumstearat | 2,5 | – |
| | 400 | 400 |

Beispiel 2

Die lipidsenkende Wirkung von N-(4,6-Dimethoxy-5-pyrimidyl)-thioharnstoff wurde an hyperlipidämischen Mäusen analog einer Untersuchungsmethode, wie in «Screening Methods in Pharmacology», Robert A. Turner, 1965, Academic Press, New York and London, und Garattini et al, Arzneimittelforschung 9, 206 (1959), angegeben, untersucht. Die Verbindung wurde in einer Menge von 10 mg/kg peroral verabreicht. Es wurde eine Senkung des Cholesterinspiegels von 25% und eine Senkung der Triglyceride von 27% erzielt.

Beispiel 3

Es wurde die lipid- und blutdrucksenkende Wirkung von N-(2,4-Dimethoxy-5-pyrimidyl)-thioharnstoff untersucht. Als Versuchstiere wurden hyperlipidämische Mäuse und spontan hypertensive Ratten verwendet.

Bei den Mäusen wurde eine Verabreichungsdosis von 100 mg/kg peroral verwendet, wobei eine Senkung des Cholesterinspiegels von 38% und eine Senkung der Triglyceride von 49% erreicht wurde.

Bei den Ratten wurde eine Dosis von 30 mg/kg peroral verabreicht, wobei eine Blutdrucksenkung von 30% erzielt wurde.

Beispiel 4

Es wurde die blutdrucksenkende Wirkung von N-(4,6-Dimethoxy-2-methyl-5-pyrimidyl)-thioharnstoff bei spontan hypertensiven Ratten untersucht. Den Ratten wurde die Verbindung in einer Dosis von 30 mg/kg peroral verabreicht. Damit wurde eine Blutdrucksenkung von 30% erzielt.

**Patentansprüche für die Vertragsstaaten: BE, CH, DE, FR, GB, IT, LI, LU, NL, SE**

1. Pyrimidylthioharnstoffe der allgemeinen Formel

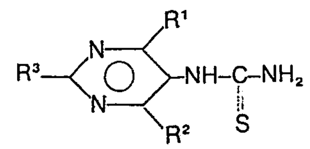

(I),

in der die Reste $R^1$, $R^2$ und $R^3$, die gleich oder verschieden sein können, ein Wasserstoff- oder Halogenatom, eine geradkettige oder verzweigte Alkoxy- oder Alkylgruppe mit jeweils 1 bis 8 Kohlenstoffatomen bedeuten oder deren physiologisch verträglichen Salze zur Anwendung als pharmazeutischer Wirkstoff.

2. Pyrimidilthioharnstoffe nach Anspruch 1 und deren physiologisch verträglichen Salze zur Anwendung bei Krankheiten des Herz-Kreislaufsystems, Hypertonie und Hyperlipidämie.

3. N-(4,6-Dimethoxy-5-pyrimidyl)-thioharnstoff,
N-(2,4-Dimethoxy-5-pyrimidyl)-thioharnstoff,
N-(4,6-Dimethoxy-2-methyl-5-pyrimidyl)-thioharnstoff und
N-(4,6-Diäthoxy-5-pyrimidyl)-thioharnstoff
gemäss Anspruch 1.

4. Pyrimidylthioharnstoffe der allgemeinen Formel

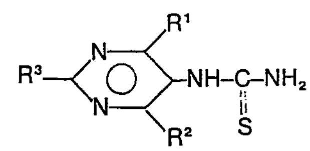

(I),

in der die Reste $R^1$, $R^2$ und $R^3$, die gleich oder verschieden sein können, ein Wasserstoff- oder Halogenatom, eine geradkettige oder verzweigte Alkoxy- oder Alkylgruppe mit jeweils 1 bis 8 Kohlenstoffatomen bedeuten, mit Ausnahme von
N-(4,6-Dimethoxy-5-pyrimidyl)-thioharnstoff,
N-(2,4-Dimethoxy-5-pyrimidyl)-thioharnstoff und
N-(4,6-Diäthoxy-5-pyrimidyl)-thioharnstoff
und deren physiologisch verträgliche Salze.

5. Pharmazeutisches Präparat, dadurch gekennzeichnet, dass es eine oder mehrere der Verbindungen gemäss Anspruch 1 und gegebenenfalls übliche Trägerstoffe und/oder Verdünnungsmittel enthält.

6. Pharmazeutisches Präparat nach Anspruch 5, dadurch gekennzeichnet, dass es als Verbindung der allgemeinen Formel I
N-(4,6-Dimethoxy-5-pyrimidyl)-thioharnstoff,
N-(2,4-Dimethoxy-5-pyrimidyl)-thioharnstoff,
N-(4,6-Dimethoxy-2-methyl-5-pyrimidyl)-thioharnstoff oder
N-(4,6-Diäthoxy-5-pyrimidyl)-thioharnstoff
oder ein physiologisch verträgliches Salz hiervon enthält.

7. Verfahren zur Herstellung der Verbindungen der allgemeinen Formel I gemäss Anspruch 1, dadurch gekennzeichnet, dass man Aminopyrimidine der allgemeinen Formel II

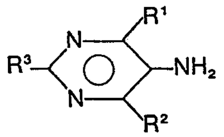

(II)

worin die Reste $R^1$, $R^2$ und $R^3$ die vorstehend angegebene Bedeutung besitzen, mit Benzoylisothiocyanat zu den entsprechenden Benzoylthioharnstoffen umsetzt und diese zu den Pyrimidylthioharnstoffen der Formel I umsetzt.

8. Verfahren zur Herstellung der Verbindungen der Formel I nach Anspruch 7, dadurch gekennzeichnet, dass man das Benzoylisothiocyanat im Reaktionsgemisch aus Ammoniumthiocyanat und Bezoylchlorid bildet.

9. Verfahren zur Herstellung der Verbindungen der Formel I nach Anspruch 7, dadurch gekennzeichnet, dass man den Benzoylrest in Gegenwart von Basen abspaltet.

10. Verfahren zur Herstellung von Thioharnstoffderivaten der allgemeinen Formel I, in der $R^1$ und $R^2$ Alkoxygruppen bedeuten und $R^3$ die vorstehend angegebene Bedeutung besitzt, dadurch gekennzeichnet, dass man Thioharnstoffe der allgemeinen Formel I, in der $R^1$ und $R^2$ Alkoxygruppen, vorzugsweise Methoxygruppen sind und $R^3$ die vorstehend angegebene Bedeutung hat, mit entsprechenden anderen Alkoholaten umsetzt.

11. Verfahren zur Herstellung von Alkoxypyrimidinen der allgemeinen Formel I, dadurch gekennzeichnet, dass man Chlorpyrimidine der allgemeinen Formel I mit einem Alkoholat umsetzt.

12. Verfahren zur Herstellung von Verbindungen der allgemeinen Formel I, in der einer oder mehrere der Reste $R^1$, $R^2$ und $R^3$ Wasserstoffatome bedeuten, dadurch gekennzeichnet, dass man Verbindungen der Formel I, in der $R^1$, $R^2$ und/oder $R^3$ Chloratome bedeuten, einer katalytischen Hydrierung unterwirft.

13. Verfahren zur Herstellung von Verbindungen der allgemeinen Formel I, in der einer oder mehrere der Reste $R^1$, $R^2$ und $R^3$ Wasserstoffatome bedeuten, dadurch gekennzeichnet, dass man Verbindungen der Formel I, in der $R^1$, $R^2$ und/oder $R^3$ Chloratome bedeuten mit Hydrazin und Silberoxid umsetzt.

**Patentansprüche für den Vertragsstaat: AT**

1. Verfahren zur Herstellung von neuen Pyrimidylthioharnstoffen, der allgemeinen Formel

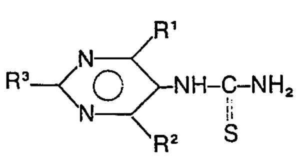

(I),

in der die Reste $R^1$, $R^2$ und $R^3$, die gleich oder verschieden sein können, ein Wasserstoff- oder Halogenatom, eine geradkettige oder verzweigte Alkoxy- oder Alkylgruppe mit jeweils 1 bis 8 Kohlenstoffatomen bedeuten, sowie deren physiologisch verträglichen Säureadditionssalze, dadurch gekennzeichnet, dass man Aminopyrimidine der allgemeinen Formel II

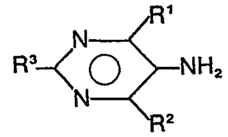

(II)

worin die Reste $R^1$, $R^2$ und $R^3$ die vorstehend angegebene Bedeutung besitzen, mit Benzoylisothiocyanat zu den entsprechenden Benzoylthioharnstoffen umsetzt und diese zu den Pyrimidylthioharnstoffen der Formel I umsetzt und gewünschtenfalls ein physiologisch verträgliches Säureadditionssalz daraus herstellt und, wenn eine Verbindung der allgemeinen Formel I wie oben definiert gewünscht wird, worin mindestens einer der Substituenten $R^1$ oder $R^2$ eine Alkoxygruppe ist und $R^3$ die angegebene Bedeutung besitzt, man eine Verbindung der Formel I, die mindestens eine Alkoxygruppe besitzt mit einem entsprechenden Alkoholat umsetzt, wobei einer oder zwei der Alkoxysubstituenten durch neue Alkoxysubstituenten ersetzt werden und, wenn eine Verbindung der allgemeinen Formel I wie oben definiert gewünscht wird, worin mindestens einer der Substituenten $R^1$, $R^2$ und $R^3$ eine Alkoxygruppe ist, man eine Verbindung der Formel I mit mindestens einem Halogenatom, mit einem entsprechenden Alkoholat umsetzt, wobei einer oder mehrere der Halogensubstituenten durch Alkoxygruppen ersetzt werden, und, wenn eine Verbindung der allgemeinen Formel I wie oben definiert gewünscht wird, worin mindestens einer der Substituenten $R^1$, $R^2$ oder $R^3$ Wasserstoff ist, man eine Verbindung der allgemeinen Formel I, die mindestens ein Halogenatom enthält, katalytisch hydriert oder mit Hydrazin und Silberoxid umsetzt, wobei einer oder mehrere der Halogensubstituenten durch Wasserstoff ersetzt werden, und gewünschtenfalls ein physiologisch verträgliches Säureadditionssalz daraus herstellt.

2. Verfahren zur Herstellung der Verbindungen der Formel I nach Anspruch 1, dadurch gekennzeichnet, dass man das Benzoylisothiocyanat im Reaktionsgemisch aus Ammoniumthiocyanat und Benzoylchlorid bildet.

3. Verfahren zur Herstellung der Verbindungen der Formel I nach Anspruch 1, dadurch gekennzeichnet, dass man den Benzoylrest in Gegenwart von Basen abspaltet.

4. Verfahren zur Herstellung von neuen Pyrimidylthioharnstoffen, der allgemeinen Formel I nach Anspruch 1,

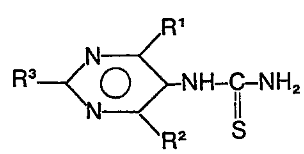

(I)

in der die Reste $R^1$, $R^2$ und $R^3$, die gleich oder verschieden sein können, ein Wasserstoff- oder Halogenatom, eine geradkettige oder verzweigte Alkoxy- oder Alkylgruppe mit jeweils 1 bis 8 Kohlenstoffatomen bedeuten, mit Ausnahme von

N-(4,6-Dimethoxy-5-pyrimidyl)-thioharnstoff,
N-(2,4-Dimethoxy-5-pyrimidyl)-thioharnstoff und
N-(4,6-Diäthoxy-5-pyrimidyl)-thioharnstoff,
sowie deren physiologisch verträglichen Säureadditionssalzen, dadurch gekennzeichnet, dass man Aminopyrimidine der allgemeinen Formel II

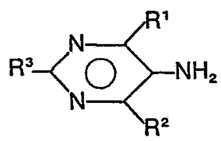

(II)

worin die Reste $R^1$, $R^2$ und $R^3$ die vorstehend angegebene Bedeutung besitzen, mit Benzoylisothiocyanat zu den entsprechenden Benzoylthioharnstoffen umsetzt und diese zu den Pyrimidylthioharnstoffen der Formel I umsetzt und gewünschtenfalls ein physiologisch verträgliches Säureadditionssalz daraus herstellt und, wenn eine Verbindung der allgemeinen Formel I wie oben definiert gewünscht wird, worin mindestens einer der Substituenten $R^1$ oder $R^2$ eine Alkoxygruppe ist und $R^3$ die angegebene Bedeutung besitzt, man eine Verbindung der Formel I, die mindestens eine Alkoxygruppe besitzt mit einem entsprechenden Alkoholat umsetzt, wobei einer oder zwei der Alkoxysubstituenten durch neue Alkoxysubstituenten ersetzt werden und, wenn eine Verbindung der allgemeinen Formel I wie oben definiert gewünscht wird, worin mindestens einer der Substituenten $R^1$, $R^2$ und $R^3$ eine Alkoxygruppe ist, man eine Verbindung der Formel I mit mindestens einem Halogenatom, mit einem entsprechenden Alkoholat umsetzt, wobei einer oder mehrere der Halogensubstituenten durch Alkoxygruppen ersetzt werden, und, wenn eine Verbindung der allgemeinen Formel I wie oben definiert gewünscht wird, worin mindestens einer der Substituenten $R^1$, $R^2$ oder $R^3$ Wasserstoff ist, man eine Verbindung der allgemeinen Formel I, die mindestens ein Halogenatom enthält, katalytisch hydriert oder mit Hydrazin und Silberoxid umsetzt, wobei einer oder mehrere der Halogensubstituenten durch Wasserstoff ersetzt werden, und gewünschtenfalls ein physiologisch verträgliches Säureadditionssalz daraus herstellt.

5. Verfahren zur Herstellung der Verbindungen der Formel I nach Anspruch 4, dadurch gekennzeichnet, dass man das Benzoylisothiocyanat im Reaktionsgemisch aus Ammoniumthiocyanat und Benzoylchlorid bildet.

6. Verfahren zur Herstellung der Verbindungen der Formel I nach Anspruch 4, dadurch gekennzeichnet, dass man den Benzoylrest in Gegenwart von Basen abspaltet.

**Claims for the Contracting States: BE, CH, DE, FR, GB, IT, LI, LU, NL, SE**

1. Pyrimidylthioureas of the general formula

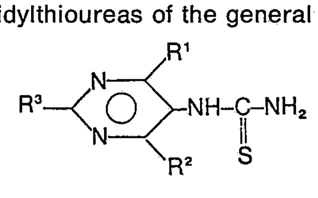

(I),

in which the radicals $R^1$, $R^2$ and $R^3$, which may be identical or different, denote a hydrogen or halogen atom or a straight-chain or branched alkoxy or alkyl group, each having 1 to 8 carbon atoms, or their physiologically tolerated salts, for use as a pharmaceutical active substance.

2. Pyrimidylthioureas according to Claim 1 and their physiologically tolerated salts, for use in disorders of the cardio-vascular system, hypertonia and hyperlipidaemia.

3. N-(4,6-Dimethoxypyrimid-5-yl)-thiourea,
N-(2,4-dimethoxypyrimid-5-yl)-thiourea,
N-(4,6-dimethoxy-2-methylpyrimid-5-yl)-thiourea and
N-(4,6-diethoxypyrimid-5-yl)-thiourea
according to Claim 1.

4. Pyrimidylthioureas of the general formula

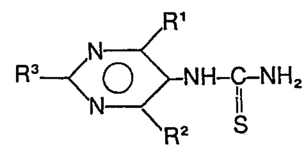

(I),

in which the radicals $R^1$, $R^2$ and $R^3$, which may be identical or different, denote a hydrogen or halogen atom or a straight-chain or branched alkoxy or alkyl group, each having 1 to 8 carbon atoms, with the exception of
N-(4,6-dimethoxypyrimid-5-yl)-thiourea,
N-(2,4-dimethoxypyrimid-5-yl)-thiourea and
N-(4,6-diethoxypyrimid-5-yl)-thiourea,
and their physiologically tolerated salts.

5. Pharmaceutical preparation, characterised in that it contains one or more of the compounds according to Claim 1 and, optionally, customary carriers and/or diluents.

6. Pharmaceutical preparation according to Claim 5, characterised in that it contains, as the compound of the general formula I,
N-(4,6-dimethoxypyrimid-5-yl)-thiourea,
N-(2,4-dimethoxypyrimid-5-yl)-thiourea,
N-(4,6-dimethoxy-2-methylpyrimid-5-yl)-thiourea or
N-(4,6-diethoxypyrimid-5-yl)-thiourea
or a physiologically tolerated salt thereof.

7. Process for the preparation of the compounds of the general formula I according to Claim 1, characterised in that aminopyrimidines of the general formula II

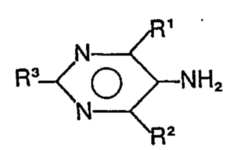

(II)

wherein the radicals $R^1$, $R^2$ and $R^3$ have the abovementioned meaning, are reacted with benzoyl isothiocyanate to give the corresponding benzoylthioureas and these are reacted to give the pyrimidylthioureas of the formula I.

8. Process for the preparation of the compounds of the formula I according to Claim 7, characterised in that the benzoyl isothiocyanate is formed from ammonia thiocyanate and benzoyl chloride, in the reaction mixture.

9. Process for the preparation of the compounds of the formula I according to Claim 7, characterised in that the benzoyl radical is split off in the presence of bases.

10. Process for the preparation of thiourea derivatives of the general formula I, in which $R^1$ and $R^2$ denote alkoxy groups and $R^3$ has the abovementioned meaning, characterised in that the thioureas of the general formula I, in which $R^1$ and $R^2$ are alkoxy groups, preferably methoxy groups, and $R^3$ has the abovementioned meaning, are reacted with appropriate other alcoholates.

11. Process for the preparation of alkoxypyrimidines of the general formula I, characterised in that chloropyrimidines of the general formula I are reacted with an alcoholate.

12. Process for the preparation of compounds of the general formula I, in which one or more of the radicals $R^1$, $R^2$ and $R^3$ denote hydrogen atoms, characterised in that compounds of the formula I, in which $R^1$, $R^2$ and/or $R^3$ denote chlorine atoms, are subjected to a catalytic hydrogenation.

13. Process for the preparation of compounds of the general formula I, in which one or more of the radicals $R^1$, $R^2$ and $R^3$ denote hydrogen atoms, characterised in that compounds of the formula I, in which $R^1$, $R^2$ and/or $R^3$ denote chlorine atoms, are reacted with hydrazine and silver oxide.

**Claims for the Contracting State: AT**

1. Process for the preparation of novel pyrimidylthioureas of the general formula

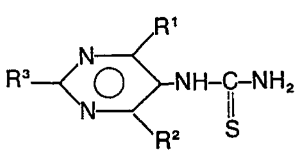 (I)

in which the radicals $R^1$, $R^2$ and $R^3$, which may be identical or different, denote a hydrogen or halogen atom or a straight-chain or branched alkoxy or alkyl group, each having 1 to 8 carbon atoms, as well as their physiologically tolerated acid addition salts, characterised in that aminopyrimidines of the general formula II

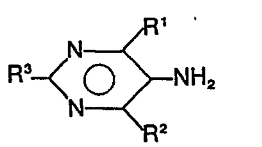 (II)

wherein the radicals $R^1$, $R^2$ and $R^3$ have the abovementioned meaning, are reacted with benzoyl isothiocyanate to give the corresponding benzoylthioureas and these are reacted to give the pyrimidylthioureas of the formula I and, if desired, a physiologically tolerated acid addition salt is prepared from these and, if a compound of the general formula I as defined above, wherein at least one of the substituents $R^1$ and $R^2$ is an alkoxy group and $R^3$ has the stated meaning, is desired, a compound of the formula I which possesses at least one alkoxy group is reacted with an appropriate alcoholate, whereby one or two of the alkoxy substituents are replaced by new alkoxy substituents and, if a compound of the general formula I as defined above, wherein at least one of the substituents $R^1$, $R^2$ and $R^3$ is an alkoxy group, is desired, a compound of the formula I which has at least one halogen atom is reacted with an appropriate alcoholate, whereby one or more of the halogen substituents are replaced by alkoxy groups, and, if a compound of the general formula I as defined above, wherein at least one of the substituents $R^1$, $R^2$ or $R^3$ is hydrogen, is desired, a compound of the general formula I, which contains at least one halogen atom, is catalytically hydrogenated or reacted with hydrazine and silver oxide, whereby one or more of the halogen substituents are replaced by hydrogen and, if desired, a physiologically tolerated acid addition salt is prepared from the product.

2. Process for the preparation of the compounds of the formula I according to Claim 1, characterised in that the benzoyl isothiocyanate is formed from ammonium thiocyanate and benzoyl chloride, in the reaction mixture.

3. Process for the preparation of the compounds of the formula I according to Claim 1, characterised in that the benzoyl radical is split off in the presence of bases.

4. Process for the preparation of novel pyrimidylthioureas of the general formula I according to Claim 1,

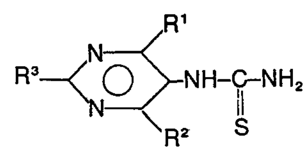 (I)

in which the radicals $R^1$, $R^2$ and $R^3$, which may be identical or different, denote a hydrogen or halogen atom or a straight-chain or branched alkoxy or alkyl group, each having 1 to 8 carbon atoms, with the exception of
N-(4,6-dimethoxypyrimid-5-yl)-thiourea,
N-(2,4-dimethoxypyrimid-5-yl)-thiourea and
N-(4,6-diethoxypyrimid-5-yl)-thiourea,
as well as their physiologically tolerated acid addition salts, characterised in that aminopyrimidines of the general formula II

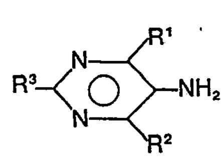 (II)

wherein the radicals $R^1$, $R^2$ and $R^3$ have the abovementioned meaning, are reacted with benzoyl isothiocyanate to give the corresponding benzoylthioureas and these are reacted to give the pyrimidylthioureas of the formula I and, if desired, a physiologically tolerated acid addition salt is prepared therefrom and, if a compound of the general formula I as defined above, wherein at least one of the substituents $R^1$ and $R^2$ is an alkoxy

group and $R^3$ has the stated meaning, is desired, a compound of the formula I which possesses at least one alkoxy group is reacted with an appropriate alcoholate, whereby one or two of the alkoxy substituents are replaced by new alkoxy substituents and, if a compound of the general formula I as defined above, wherein at least one of the substituents $R^1$, $R^2$ and $R^3$ is an alkoxy group, is desired, a compound of the formula I which has at least one halogen atom is reacted with an appropriate alcoholate, whereby one or more of the halogen substituents are replaced by alkoxy groups, and, if a compound of the general formula I as defined above, wherein at least one of the substituents $R^1$, $R^2$ or $R^3$ is hydrogen, is desired, a compound of the general formula I, which contains at least one halogen atom, is catalytically hydrogenated or reacted with hydrazine and silver oxide, whereby one or more of the halogen substituents are replaced by hydrogen and, if desired, a physiologically tolerated acid addition salt is prepared from the product.

5. Process for the preparation of the compounds of the formula I according to Claim 4, characterised in that the benzoyl isothiocyanate is formed from ammonium thiocyanate and benzoyl chloride, in the reaction mixture.

6. Process for the preparation of the compounds of the formula I according to Claim 4, characterised in that the benzoyl radical is split off in the presence of bases.

**Revendications pour les Etats Contractants: BE, CH, DE, FR, GB, IT, LI, LU, NL, SE**

1. Pyrimidylthiourées de formule générale

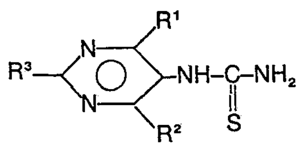

(I),

dans laquelle les radicaux $R^1$, $R^2$ et $R^3$, qui peuvent être identiques ou différents, désignent un atome d'hydrogène ou d'un halogène ou un groupe alcoxy ou alkyle à chaîne droite ou ramifiée, chacun possédant 1 à 8 atomes de carbone, ou leurs sels physiologiquement tolérés, destinées à être utilisées comme substance pharmaceutique active.

2. Pyrimidylthiourées selon la revendication 1 et leurs sels physiologiquement tolérés, destinées à être utilisées pour les troubles du système cardiovasculaire, l'hypertonie et l'hyperlipidémie.

3. N-(Diméthoxy-4,6 pyrimidyl-5)-thiourée,
N-(diméthoxy-2,4 pyrimidyl-5)-thiourée,
N-(diméthoxy-4,6 méthyl-2 pyrimidyl-5)-thiourée et
N-(diéthoxy-4,6 pyrimidyl-5)-thiourée
selon la revendication 1.

4. Pyrimidylthiourées de formule générale

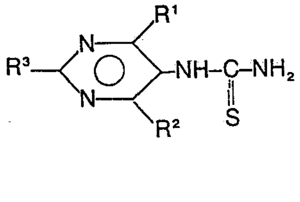

(I),

dans laquelle les radicaux $R^1$, $R^2$ et $R^3$, qui peuvent être identiques ou différents, désignent un atome d'hydrogène ou d'un halogène ou un groupe alcoxy ou alkyle à chaîne droite ou ramifiée, chacun possédant 1 à 8 atomes de carbone, à l'exception de
la N-(diméthoxy-4,6 pyrimidyl-5)-thiourée,
la N-(diméthoxy-2,4 pyrimidyl-5)-thiourée et
la N-(diéthoxy-4,6 pyrimidyl-5)-thiourée,
et leurs sels physiologiquement tolérés.

5. Préparation pharmaceutique, charactérisée en ce qu'elle contient un ou plusieurs des composés selon la revendication 1 et, éventuellement, des véhicules et/ou des diluants classiques.

6. Préparation pharmaceutique selon la revendication 5, caractérisée en ce qu'elle contient, comme le composé de formule générale I,
la N-(diméthoxy-4,6 pyrimidyl-5)-thiourée,
la N-(diméthoxy-2,4 pyrimidyl-5)-thiourée,
la N-(diméthoxy-4,6 méthyl-2-pyrimidyl-5)-thiourée ou
la N-(diéthoxy-4,6 pyrimidyl-5)-thiourée
ou un de leurs sels physiologiquement toléré.

7. Procédé de préparation des composés de formule générale I selon la revendication 1, caractérisé en ce que l'on fait réagir des aminopyrimidines de formule générale II

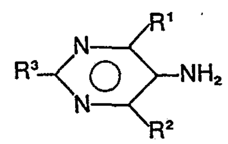

(II)

dans laquelle les radicaux $R^1$, $R^2$ et $R^3$ ont les significations mentionées ci-dessus, avec l'isothiocyanate de benzoyle pour donner les benzoylthiourées correspondantes et en ce que l'on fait réagir celles-ci pour donner les pyrimidylthiourées de formule I.

8. Procédé de préparation des composés de formule I selon la revendication 7, caractérisé en ce que l'isothiocyanate de benzoyle est formé à partir de thiocyanate d'ammonium et de chlorure de benzoyle, dans le mélange réactionnel.

9. Procédé de préparation des composés de formule I selon la revendication 7, caractérisé en ce que le radical benzoyle est séparé en présence de bases.

10. Procédé de préparation de dérivés de thiourée de formule générale I, dans laquelle $R^1$ et $R^2$ désignent des groupes alcoxy et $R^3$ a la signification mentionnée ci-dessus, caractérisé en ce que l'on fait réagir es thiourées de formule générale I, dans laquelle $R^1$ et $R^2$ sont des groupes alcoxy, de préférence des groupes méthoxy, et $R^3$ a la signification mentionnée ci-dessus, avec d'autres alcoolates appropriés.

11. Procédé de préparation d'alcoxypyrimidines de formule générale I, caractérisé en ce que l'on fait réagir les chloropyrimidines de formule générale I avec un alcoolate.

12. Procédé de préparation de composés de formule générale I, dans laquelle un ou plusieurs des radicaux $R^1$, $R^2$ et $R^3$ désignent des atomes d'hydrogènes, caractérisé en ce que des composés de formule I, dans lesquels $R^1$, $R^2$ et/ou $R^3$ désignent des atomes de chlore, sont soumis à une hydrogénation catalytique.

13. Procédé de préparation de composés de formule générale I, dans laquelle un ou plusieurs des radicaux $R^1$, $R^2$ et $R^3$ désignent des atomes d'hydrogène, caractérisé en ce que l'on fait réagir des composés de formule I, dans laquelle $R^1$, $R^2$ et/ou $R^3$ désignent des atomes de chlore, avec de l'hydrazine et de l'oxyde d'argent.

**Revendications pour l'Etat Contractant: AT**

1. Procédé de préparation de nouvelles pyrimidylthiourées de formule générale

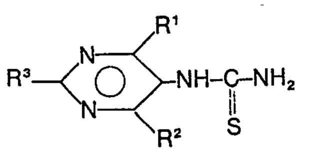

(I)

dans laquelle les radicaux $R^1$, $R^2$ et $R^3$, qui peuvent être identiques ou différents, désignent un atome d'hydrogène ou d'halogène ou un groupe alcoxy ou alkyle à chaîne droite ou ramifiée, chacun possédant 1 à 8 atomes de carbone, ainsi que leurs sels d'addition avec des acides physiologiquement tolérés, caractérisé en ce que l'on fait réagir des aminopyrimidines de formule générale II

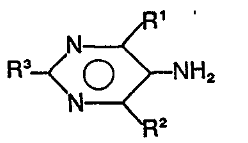

(II)

dans laquelle les radicaux $R^1$, $R^2$ et $R^3$ ont les significations mentionnées ci-dessus, avec l'isothiocyanate de benzoyle pour donner les benzoylthiourées correspondantes et en ce que l'on fait réagir celles-ci pour donner les pyrimidylthiourées de formule I et, éventuellement, on prépare à partir de celles-ci un sel d'addition avec un acide physiologiquement toléré et, si l'on désire un composé de formule générale I telle que définie ci-dessus, dans laquelle l'un au moins des substituants $R^1$ et $R^2$ est un groupe alcoxy et $R^3$ a la signification indiquée, on fait réagir un composé de formule I qui possède au moins un groupe alcoxy avec un alcoolate approprié, réaction dans laquelle un ou deux des substituants alcoxy sont remplacés par de nouveaux substituants alcoxy et, si l'on désire un composé de formule générale I telle que définie ci-dessus, dans laquelle l'un au moins des substituants $R^1$, $R^2$ et $R^3$ est un groupe alcoxy, on fait réagir un composé de formule I qui possède au moins un atome d'un halogène avec un alcoolate approprié réaction dans laquelle un ou plusieurs des substituants halogène sont remplacés par des groupes alcoxy et, si l'on désire un composé de formule générale I telle que définie ci-dessus, dans laquelle l'un au moins des substituants $R^1$, $R^2$ et $R^3$ est l'hydrogène, on effectue une hydrogénation catalytique d'un composé de formule générale I qui possède au moins un atome d'un halogène ou on le fait réagir avec de l'hydrazine et de l'oxyde d'argent, réaction dans laquelle un ou plusieurs des substituants halogène sont remplacés par l'hydrogène et, éventuellement, on prépare, à partir de ce produit, un sel d'addition avec un acide physiologiquement toléré.

2. Procédé de préparation des composés de formule I selon la revendication 1, caractérisé en ce que l'isothiocyanate de benzoyle est formé à partir de thiocyanate d'ammonium et de chlorure de benzoyle, dans le mélange réactionnel.

3. Procédé de préparation des composés de formule I selon la revendication 1, caractérisé en ce que le radical benzoyle est séparé en présence de bases.

4. Procédé de préparation de nouvelles pyrimidylthiourées de formule générale I selon la revendication 1,

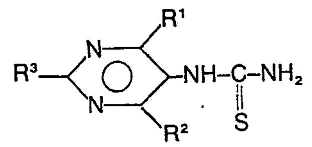

(I)

dans laquelle les radicaux $R^1$, $R^2$ et $R^3$, qui peuvent être identiques ou différents, désignent un atome d'hydrogène ou d'un halogène ou un groupe alcoxy ou alkyle à chaîne droite ou ramifiée, chacun ayant 1 à 8 atomes de carbone, à l'exception de la N-(diméthoxy-4,6 pyrimidyl-5)-thiourée, la N-(diméthoxy-2,4 pyrimidyl-5)-thiourée et la N-(diéthoxy-4,6 pyrimidyl-5)-thiourée, ainsi que leurs sels d'addition avec des acides physiologiquement tolérés, caractérisé en ce que l'on fait réagir des aminopyrimidines de formule générale II

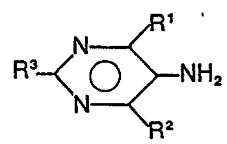

(II)

dans laquelle les radicaux $R^1$, $R^2$ et $R^3$ ont les significations mentionnées ci-dessus, avec l'isothiocyanate de benzoyle pour donner les benzoylthiourées correspondantes et en ce que l'on fait réagir celles-ci pour donner les pyrimidylthiourées de formule I et, éventuellement, on prépare à partir de celles-ci un sel d'addition avec un acide physiologiquement toléré et, si l'on désire un composé de formule générale I telle que définie ci-dessus, dans laquelle l'un au moins des substituants $R^1$ et $R^2$ est un groupe alcoxy et $R^3$ a la signification indiquée, on fait réagir un composé de formule I qui possède au moins un groupe alcoxy avec un alcoolate approprié, réaction dans laquelle un ou deux des substituants alcoxy sont remplacés par de nouveaux substituants alcoxy et, si l'on désire un composé de formule générale I telle que définie ci-dessus, dans laquelle l'un au moins des substituants $R^1$, $R^2$ et $R^3$ est un groupe alcoxy, on fait réagir un composé de formule I qui possède au moins un atome d'un halogène avec

un alcoolate approprié, réaction dans laquelle un ou plusieurs des substituants halogène sont remplacés par des groupes alcoxy et, si l'on désire un composé de formule générale I telle que définie ci-dessus, dans laquelle l'un au moins des substituants $R^1$, $R^2$ et $R^3$ est l'hydrogène, on effectue une hydrogénation catalytique d'un composé de formule générale I qui possède au moins un atome d'un halogène ou on le fait réagir avec de l'hydrazine et de l'oxyde d'argent, réaction dans laquelle un ou plusieurs des substituants halogène sont remplacés par l'hydrogène et, éventuellement, on prépare, à partir de ce produit, un sel d'addition avec un acide physiologiquement toléré.

5. Procédé de préparation des composés de formule I selon la revendication 4, caractérisé en ce que l'isothiocyanate de benzoyle est formé à partir de thiocyanate d'ammonium et de chlorure de benzoyle, dans le mélange réactionnel.

6. Procédé de préparation des composés de formule I selon la revendication 4, caractérisé en ce que le radical benzoyle est séparé en présence de bases.